# FASCICULE DE BREVET EUROPEEN

(11) **EP 1 424 058 B1**
(45) Date de publication et mention de la délivrance du brevet: **18.07.2007**
(21) Numéro de dépôt: 03292109.0
(22) Date de dépôt: 27.08.2003
(51) Int. Cl.: A61K 8/25, A61K 8/31, A61K 8/37, A61K 8/73, A61K 8/81, A61K 8/88, A61K 8/92, A61Q 1/10

(54) **Procédé de maquillage des cils avec une composition comprenant une cire collante.**
Verfahren zur dekorativen Behandlung der Wimpern mit einem Überzugsmittel, das ein klebriges Wachs enthält
Make-up process for eyelashes with a composition comprising a tacky wax.

(30) Priorité: 06.09.2002 FR 0211096; 06.09.2002 FR 0211104; 06.09.2002 FR 0211097; 30.09.2002 FR 0212097; 30.09.2002 FR 0212098
(43) Date de publication de la demande: 02.06.2004
(73) Titulaire: L'ORÉAL, 75008 Paris (FR)
(72) Inventeur: De La Poterie, Valérie, 77820 Le Chaletel en Brie (FR); Daubige, Thérèse, 77480 Mousseaux les Bray (FR); Styczen, Patrice, 91190 Gif-sur-Yvette (FR)
(74) Mandataire: Kromer, Christophe

(56) Documents cités:
- EP-A- 0 987 002
- EP-A- 0 998 905
- EP-A- 1 080 713
- WO-A-91/12793
- DE-A- 19 751 221
- JP-A- 10 139 631
- US-A- 5 783 176

## Description

La présente invention a pour objet un procédé cosmétique de maquillage ou de traitement des cils comprenant l'application sur les cils d'une composition comprenant une cire collante

La composition du procédé selon l'invention peut être une composition de maquillage, encore appelée mascara, une base de maquillage des cils ou base-coat, une composition à appliquer sur un maquillage, dite encore top-coat, ou bien encore une composition de traitement des cils dite encore basecoat. Plus spécialement, la composition selon l'invention est un mascara.

Par mascara, on entend une composition destinée à être appliquée sur les cils. Le mascara est plus particulièrement destiné aux cils d'êtres humains, mais également aux faux-cils.

Les mascaras sont couramment préparés selon deux types de formulation : les mascaras aqueux, dits mascaras crèmes, sous forme d'émulsion de cires dans l'eau ; les mascaras anhydres ou à faible teneur en eau, dits mascaras water-proofs, sous forme de dispersions de cires dans des solvants organiques.
Il est connu d'employer diverses cires pour la formulation des mascaras comme celles décrites dans le document WO-A-91/12793, par exemple la cire d'abeille, la cire de candellila, la cire de carnauba, ou bien encore la cire de polyéthylène.

Toutefois, lorsque les mascaras contiennent certaines cires comme la cire de carnauba, la cire de son de riz ou la cire de polyéthylène, le maquillage des cils obtenu présente un aspect granuleux conférant ainsi un maquillage non lisse et non homogène, défauts qui rendent le maquillage inesthétique.

Par ailleurs, pour obtenir un mascara présentant de bonnes propriétés chargeantes, c'est-à-dire obtenir un maquillage épais des cils, il est possible d'incorporer dans le mascara une ou plusieurs cires en une teneur totale supérieure à 25 % en poids du poids total du mascara. Or en utilisant les cires classiques comme la cire d'abeille, la cire de candellila, ou la cire de carnauba dans ces teneurs élevées, la composition de mascara devient très consistante, voire trop compacte, et ne peut pas être appliquée facilement sur les cils avec les applicateurs de brosse à mascara couramment utilisés. Le mascara trop épais est déposé sur les cils sous forme de paquets et le maquillage ainsi obtenu ne présente pas l'aspect lisse recherché; le maquillage n'est pas homogène et a un aspect inesthétique. En outre, certaines cires comme la cire d'orange, la cire de lanoline, employées à des teneurs supérieures à 25 % en poids conduisent à des compositions qui ne sont pas suffisamment stables, notamment après un stockage de deux semaines à température ambiante (25 °C); la composition prend en masse (augmentation importante de la viscosité) ou présente un déphasage qui s'observe visuellement à l'oeil nu. La composition est alors inappropriée pour être appliquée sur les cils.

Le but de la présente invention est de proposer un procédé de revêtement des cils permettant d'obtenir un maquillage des cils homogène et lisse.

Un autre but de la présente invention est de disposer d'un procédé de revêtement des cils comprenant l'application d'une composition qui s'applique facilement sur les cils et permettant un maquillage rapide desdits cils, pouvant comprendre un taux élevé de cire et présentant de bonnes propriétés chargeantes des cils. Un autre but de l'invention est d'obtenir un procédé comprenant l'application d'une composition de revêtement des fibres cils qui soit stable, notamment après un stockage de 24 heures à 25 °C.

Les inventeurs ont découverts qu'une telle composition pouvait être obtenue en utilisant une cire particulière présentant des propriétés collantes (collant élevé). Cette cire permet et d'obtenir un mascara qui s'applique facilement sur les cils, ayant une bonne accroche sur les cils et qui permet une application rapide du maquillage et conduit à la formation d'un maquillage lisse et homogène, ne présentant pas d'aspect granuleux.
De plus, la cire collante peut être incorporée dans la composition en une teneur pouvant aller jusqu'à 60 % en poids, par rapport au poids total de la composition, sans obtenir une prise en masse de la composition: la composition est stable (notamment stabilité après 24 heures à 25 °C), reste d'une consistance crémeuse et s'applique facilement sur les cils.
Par ailleurs, le mascara permet d'obtenir une bonne séparation des cils : ces der niers ne sont pas collés entre eux.

De façon plus précise, l'invention a pour objet un procédé cosmétique de maquillage ou de soin non thérapeutique des cils comprenant l'application sur les cils d'une composition comprenant, dans un milieu cosmétiquement acceptable, une première cire ayant un collant supérieur ou égal à 0,7 N.s et une dureté inférieure ou égale à 3,5 MPa, le collant étant mesuré à 20 °C à l'aide du texturomètre vendu sous la dénomination TA-TX2i par la société RHEO, équipé d'un mobile en polymère acrylique en forme de cône formant un angle de 45°, selon le protocole indiqué plus loin, et la dureté étant mesurée à 20 °C à l'aide du texturomètre vendu sous la dénomination TA-XT2i- par la société RHEO, équipé d'un cylindre en inox d'un diamètre de 2 mm, selon le protocole indiqué plus loin.

On entend par « milieu cosmétiquement acceptable » un milieu cosmétique compatible avec les cils ou la peau.

La première cire, appelée aussi cire collante, présente dans la composition selon l'invention a un collant supérieur ou égal à 0,7 N.s, notamment allant de 0,7 N,s à 30 N.S, de préférence supérieur ou égal à 1 N.s , notamment allant de 1 N.s à 20 N.s, et préférentiellement supérieur ou égal à 2 N.s, notamment allant de 2 N.s à 10 N.s, notamment, encore mieux allant de 2 N.s à 5 N.s.

La cire collante a une dureté inférieure ou égale à 3,5 MPa, en particulier allant de 0.01 à 3.5 MPa, de préférence allant de 0,05 MPa à 3 MPa, et plus préférentiellement allant de 0,1 MPa à 2,5 MPa.

Par "cire", on entend au sens de la présente invention, un composé gras lipophile, solide à température ambiante (25°C) et pression atmosphérique (760 mm de Hg, soit 10⁵ Pa), à changement d'état solide/liquide réversible, ayant une température de fusion supérieure à 30°C et mieux supérieure à 55 °C et pouvant aller jusqu'à 200° C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Le collant de la première cire est mesuré à 20 °C à l'aide du texturomètre vendu sous la dénomination TA-TX2i par la société RHEO, équipé d'un mobile en polymère acrylique en forme de cône formant un angle de 45°, en mesurant l'évolution de la force (force de compression ou force d'étirement) (F) en fonction du temps, pendant l'opération suivante :
Le mobile est déplacé à la vitesse de 0,5 mm /s puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

Pour effectuer la mesure du collant de la cire, la cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

La dureté de la première cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression maximale mesurée divisée par la surface du cylindre du texturomètre en contact avec la cire.

Comme cire collante, on peut utiliser un (hydroxystéaroyloxy)stéarate d'alkyle en C₂₀-C₄₀ (le groupe alkyle comprenant de 20 à 40 atomes de carbone), seul ou en mélange, en particulier un 12-(12'-hydroxystéaroyloxy)stéarate d'alkyle en C₂₀-C₄₀, de formule (I) : 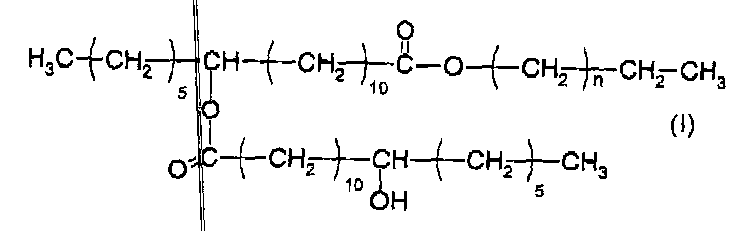 dans laquelle n est un entier allant de 18 à 38, ou un mélange de composés de formule (1).

Une telle cire collante est notamment vendue sous les dénominations « KESTER WAX K 82 P » et « KESTER WAX K 80 P » par la société KOSTER KEUNEN.

La première cire peut se présenter sous la forme d'une microdispersion aqueuse de particules de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

La cire collante peut être présente dans la composition selon l'invention en une teneur allant de 0,5 % à 60 % en poids, par rapport au poids total de la composition, en particulier en une teneur supérieure ou égale à 0,5 % et inférieure à 25 % en poids, de préférence allant de 5 % à 50 % en poids, et plus préférentiellement allant de 10 % à 40 % en poids.

En particulier la cire collante peut être présente dans la composition selon l'invention en une teneur supérieure à 25 % en poids par rapport au poids total de la composition, par exemple allant de 25 à 60 % en poids, de préférence supérieure à 27% en poids, par exemple de 27 à 50 % en poids, mieux supérieure à 28% en poids, par exemple allant de 28 à 45 % en poids et plus préférentiellement supérieure à 30 % en poids, par exemple de 30 à 40% en poids.

### Deuxième cire

Avantageusement, la composition selon l'invention comprend une deuxième cire, appelée aussi cire dure, qui a une dureté supérieure ou égale à 6 MPa , notamment allant de 6 MPa à 30 MPa, et de préférence supérieure ou égale à 7 MPa, notamment allant de 7 MPa à 25 MPa et, mieux supérieure ou égale à 8 MPa , notamment de 8 à 25 MPa, encore mieux supérieure ou égale à 9 MPa, par exemple de 9 à 20 MPa et plus préférentiellement supérieure ou égale à 10 MPa, notamment de 10 à 20 MPa.

La dureté de la cire dure est mesurée selon le même protocole décrit précédemment pour la première cire.

Comme cire dure, on peut utiliser la cire de Carnauba, de Candellila, les cires de polyéthylène, l'huile de jojoba hydrogénée, la cire de sumac, la cérésine, le stéarate d'octacosanyle, le stéarate de tétracontanyle, la cire de Shellac, le fumarate de béhényle, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE, les ozokerites comme celle vendue sous la dénomination « OZOKERITE WAX SP 1020 P » par la société STRAHL & PITSCH la cire obtenue par hydrogénation d'huile d'olive estérifiée avec l'alcool stéarylique vendue sous la dénomination PHYTOWAX Olive 18 L 57 par la Société SOPHIM.

La deuxième cire peut se présenter sous forme de microdispersion aqueuse de-cire comme décrit précédemment pour la première cire.

La deuxième cire dure peut être présente dans la composition selon l'invention en une teneur allant de 0,1 % à 30 % en poids, par rapport au poids total de la composition, de préférence allant de 1% à 20 % en poids, et plus préférentiellement allant de 2 % à 10 % en poids.

Pour certaines applications cosmétiques, il serait avantageux de pouvoir modérer le collant naturel de la première cire, notamment lorsqu'elle est présente en quantité importante (typiquement supérieure à 10%, mieux 20% et mieux 25%) tout en conservant les propriétés avantageuses de dépot lisse et homogène. Ainsi, dans le cas particulier des formules de type mascara, certaines utilisatrices cherchent à obtenir une parfaite individualisation des cils qui n'est pas toujours optimale en présence d'une cire collante.
D'une manière inattendue, les inventeurs ont constaté qu'il était possible de satisfaire à cette exigence supplémentaire sous réserve d'associer à ladite cire collante au moins un composé choisi parmi un ester de dextrine et d'acide(s) gras et/ou une charge possédant une surface spécifique BET supérieure ou égale à 100 m²/g.

Avantageusement, la composition selon l'invention comprend au moins un composé choisi parmi un ester de dextrine et d'acide(s) gras et/ou une charge possédant une surface spécifique BET supérieure ou égale à 100 m²/g.

### Charge à surface spécifique

La charge peut posséder une surface spécifique supérieure ou égale à 100 m²/g, notamment variant de 100 à 5000 m²/g, en particulier de 150 à 1000 m²/g, voire de 200 à 800 m²/g.

Par « charge », on désigne des particules de toutes formes, insolubles dans le milieu de la composition, quelle que soit la température à laquelle la composition est fabriquée.
Par « charge à surface spécifique », on entend une charge possédant une surface spécifique mesurée selon la méthode BET supérieure ou égale à 100 m²/g.

La « surface spécifique BET » est déterminée selon la méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale (donc micropores compris) de la charge.

La quantité en charge dans la composition selon l'invention est généralement ajustée de manière à contrôler le collant de la cire associée, à la valeur souhaitée. Ainsi, les charges conformes à l'invention sont particulièrement avantageuses pour la préparation de compositions cosmétiques comprenant au moins 10 %, notamment 20 %, en particulier au moins 25 %, et mieux 27% en poids d'au moins une cire collante.

A titre illustratif, cette charge peut être présente dans la composition selon l'invention en une quantité allant de 0,1 à 25 %, notamment de 0,5 à 20 % et en particulier de 1 à 15 % en poids par rapport au poids total de la composition.

Avantageusement, la première cire (cire collante) et la charge à surface spécifique sont présentes en une teneur telle que le rapport pondéral de la première cire par rapport à la charge à surface spécifique varie de 350 à 0,1, notamment de 100 à 0,5, en particulier de 50 à 0,8 et mieux de 30 à 1.

Les particules composant la charge à surface spécifique peuvent avoir une taille moyenne variant de 0,01 à 100 µm, notamment de 0,1 à 50 µm, et mieux de 1 à 20 µm. Par « taille moyenne », on désigne la dimension donnée par la distribution granulométrique statistique à la moitié de la population, dites D50.

La charge à surface spécifique conforme à l'invention peut être notamment choisie parmi les charges organiques, minérales et/ou leurs mélanges.
La charge organique peut être choisie parmi les cires polyoléfiniques comme les cires de polyéthylène et notamment celles commercialisées sous la dénomination « Performalen 2000^{®} » par la société New Phase Technology ou parmi les charges polymériques telles que le polymethylmethacrylate (PMMA) comme le Jurymer MB1^{®} vendu par Nihon Junyaku ou le polytétrafluoroéthylène (PTFE). A titre représentatif et non limitatif des charges minérales, on peut notamment citer les silices, les silicates, les alumines, les aluminosilicates, notamment celles commercialisées sous la dénomination « Sunsil 130^{®} » par la Société Sunjin Chemical ou « Silicabeads SB150^{®} »par la Société Miyoshi.

Les particules composant la charge à surface spécifique peuvent présenter des formes variées. Ces particules peuvent être notamment globulaires, lamellaires, en particulier sphériques, creuses ou pleines. Les charges creuses s'avèrent particulièrement avantageuses.
Ainsi, dans le cas particulier d'une charge minérale, conviennent tout particulièrement les microsphères de silice creuse, et en particulier telles que le « Sunsphère H-51 » d'Asahi Glass de surface spécifique égale à 770 m²/g, et le « Sunsil 130 » de Sunjin Chemical de surface spécifique 200-260 m²/g.

Selon un mode de réalisation particulier de l'invention, la composition comprend au moins une première cire (cire collante) et au moins une charge possédant une surface spécifique supérieure ou égale à 100 m²/g, telle que de la silice creuse et sphérique, des microsphères de silice creuse, et/ou de la cire de polyéthylène.

### Ester de dextrine et d'acide(s) gras

L'ester de dextrine et d'acide(s) gras qui peut être associé à la cire collante dans la composition selon l'invention est plus particulièrement, un mono-ou poly-ester de dextrine et d'au moins un acide gras et notamment répondant à la formule (II) : 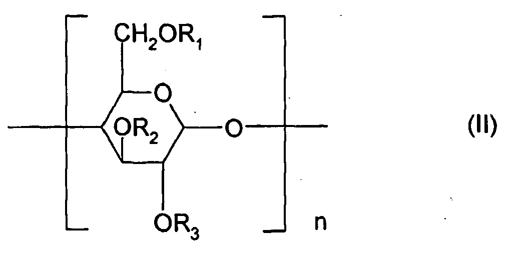 dans laquelle :
- n est un entier allant de 3 à 200, notamment allant de 20 à 150, et en particulier allant de 25 à 50,
- les radicaux R_{1,} R₂ et R_{3,} identiques ou différents, sont choisis parmi l'hydrogène ou un groupement acyle (R-CO-) dans lequel le radical R est un groupement hydrocarboné, linéaire ou ramifié, saturé ou insaturé, possédant de 6 à 50 en particulier de 7 à 21, notamment de 11 à 19, plus particulièrement de 13 à 17, voire 15, atomes de carbone, sous réserve qu'au moins un desdits radicaux R₁, R₂
ou R₃ est différent de l'hydrogène.
En particulier, R_{1,} R₂ et R₃ peuvent représenter l'hydrogène ou un groupement acyle (R-CO-) dans lequel R est un radical hydrocarboné tel que défini précédemment, sous réserve qu'au moins deux desdits radicaux R_{1,} R₂ ou R₃ sont identiques et différents de l'hydrogène.
L'ensemble des radicaux R₁, R₂ et R₃ peuvent figurer un groupement acyle (R-CO) identique ou différent et notamment identique.
En particulier, n varie avantageusement de 25 à 50, notamment est égal à 38 dans la formule générale (II) de l'ester selon l'invention.
Notamment lorsque les radicaux R₁, R₂ et/ou R₃, identiques ou différents figurent un groupement acyle (R-CO), ceux-ci peuvent être choisis parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine, en particulier des palmitates de dextrine, sont disponibles commercialement sous la dénomination RHEOPEARL TL ou RHEOPEARL KL de la société Chiba Flour.

L'ester de dextrine peut être présent dans la composition selon l'invention à une quantité allant de 0,1 à 20 %, en particulier de 0,5 à 15 % en poids, et notamment de 1 à 10 % en poids, par rapport au poids total de la composition.

Avantageusement, la première cire (cire collante) et l'ester de dextrine sont présents en une teneur telle que le rapport pondéral de la première cire par rapport à l'ester de dextrine varie de 350 à 0,1, en particulier de 100 à 0,5, notamment de 50 à 1, voire de 15 à 2.

### Milieu cosmétiquement acceptable

Le milieu cosmétiquement acceptable de la composition peut comprendre un solvant volatil, notamment choisi parmi l'eau, les solvants organiques volatils et les huiles volatiles définis ci-après, et leurs mélanges.

La composition selon l'invention peut comprendre un milieu aqueux, constituant une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.
La phase aqueuse (eau et éventuellement le solvant organique miscible à l'eau) peut être présente, en une teneur allant de 1 % à 95 % en poids, par rapport au poids total de la composition, de préférence allant de 3 % à 80 % en poids, et préférentiellement allant de 5 % à 60 % en poids.

La composition selon l'invention peut comprendre une huile ou solvant organique qui peut notamment former une phase grasse, et en particulier une phase grasse continue. La composition peut être une composition anhydre.

Par " huile ou solvant organique volatile", on entend au sens de l'invention tout milieu non aqueux susceptible de s'évaporer au contact de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg) . Par "huile non volatile", on entend une huile restant sur la fibre kératinique à température ambiante et pression atmosphérique au moins plusieurs heures et ayant notamment une pression de vapeur inférieure à 10⁻³ mm de Hg (0,13Pa).
Ces huiles peuvent être des huiles hydrocarbonées, des huiles siliconées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 6 centistokes (6 10⁻⁶ m²/s), et ayant notamment de 2 à 10 atomes de silicium, ces silicones comportant éventuellement des groupes alkyle ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C_{24,} ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles non volatiles peuvent être présentes dans la composition selon l'invention en une teneur allant de 0 à 30 % (notamment de 0,1 à 30 %) en poids, de préférence de 0 % à 20 % en poids (notamment 0,1 à 20 %), par rapport au poids total de la composition, et mieux de 0 % à 10 % en poids (notamment 0,1 % à 10 %).

La composition selon l'invention peut comprendre en outre une cire additionnelle, différente de la première cire (cire collante) et de la deuxième cire (cire dure) décrites précédemment.

La cire additionnelle peut être choisie par exemple parmi la cire d'abeille, les cires de paraffine, l'huile de ricin hydrogénée, les cires de silicone.

La cire additionnelle peut être également être présente sous forme de microdispersion de cire telle que décrite plus haut pour la première cire et la deuxième cire.

La cire additionnelle peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La teneur totale en cires (première cire et/ou seconde cire et/ou cire additionnelle) de la composition selon l'invention peut aller de 0,7 à 70% en poids par rapport au poids total de la composition, de préférence de 5 à 65% en poids, mieux de 10 à 60% en poids, et encore mieux de 15 à 50% en poids.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0,01 à 60% en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un polymère filmogène.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C_{30,} de préférence en C₁-C_{20,} des (méth)acrylates d'aryle, en particulier d'aryle en C₆-C₁₀, des (méth)acrylates d'hydroxyalkyle, en particulier d'hydroxyalkyle en C₂-C₆ .
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxyliqué. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoé-thanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est dons solubilisé dans la phase aqueuse de la composition. Comme exemples de polymères filmogènes hydrosolubles, on peut citer:
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   - les gommes arabiques, la gomme de guar, les dérivés du xanthane, la gomme de karaya ;
   - les alginates et les carraghénanes ;
   - les glycoaminoglycanes, l'acide hyaluronique et ses dérivés ;
   - la résine shellac, la gomme de sandaraque, les dammars, les élémis, les copals ;
   - l'acide désoxyribonucléïque ;
   - les muccopolysaccharides tels les chondroïtines sulfate,
et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par "phase grasse liquide", on entend, au sens de l'invention, une phase grasse liquide à température ambiante (25°C) et pression atmosphérique (760 mm de Hg, soit 10⁵ Pa), composée d'un ou plusieurs corps gras liquides à température ambiante, appelés aussi huiles, généralement compatibles entre eux.
De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, polystéarate de vinyle réticulé à l'aide de divinylbenzène, de diallyléther ou de phtalate de diallyle, poly(méth)acrylate de stéaryle, polylaurate de vinyle, poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C_{20,} comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations « Neocryl XK-90^{®} », « Neocryl A-1070^{®} », « Neocryl A-1090^{®} », « Neocryl BT-62^{®} », « Neocryl A-1079^{®} » et « Neocryl A-523^{®} » par la société AVECIA-NEORESINS, « Dow Latex 432^{®} » par la société DOW CHEMICAL, « Daitosol 5000 AD^{®} » par la société DAITO KASEY KOGYO; ou encore les dispersions aqueuses de polyuréthane vendues sous les dénominations « Neorez R-981^{®} » et « Neorez R-974^{®} » par la société AVECIA-NEORESINS, les « Avalure UR-405^{®} », « Avalure UR-410^{®} », « Avalure UR-425^{®} », « Avalure UR-450^{®} », « Sancure 875^{®} », « Sancure 861^{®} », « Sancure 878^{®} » et « Sancure 2060^{®} » par la société GOODRICH, « Impranil 85^{®} » par la société BAYER, « Aquamere H-1511^{®} » par la société HYDROMER ; les sulfopolyesters vendus sous le nom de marque « Eastman AQ^{®} » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### Additifs

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition de l'invention peut comprendre, en outre, tout additif usuellement utilisé en cosmétique tels que les antioxydants, les charges autres que la charge à surface spécifique décrite plus haut, les conservateurs, les parfums, les neutralisants, les épaississants, les vitamines, et leurs mélanges.

De préférence, la composition selon l'invention est un mascara.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

La composition selon l'invention peut être conditionnée dans un produit d'application comprenant un réservoir et un moyen amovible pour fermer, de préférence de manière étanche, ledit réservoir.
Ledit ensemble d'application peut comprendre en outre un organe d'application de la composition de maquillage sur les cils, ledit organe d'application permettant le prélèvement de la composition et la restitution de la composition prélevée sur les cils. Cet organe d'application est de préférence solidaire des moyens de fermeture étanche de l'ensemble.
L'ensemble d'application peut également comprendre un organe d'essorage (ou essoreur) dudit organe d'application, l'organe d'essorage pouvant être solidaire du réservoir.

L'organe d'application peut être de préférence une brosse à mascara bien connue de l'homme du métier. Une telle brosse comprend notamment des poils disposés radialement autour d'une âme torsadée, en particulier une âme métallique. La brosse peut être de forme variée et comporter des découpes. Des brosses à mascara sont par exemple décrites dans les documents FR-A-2607373, EP-A-611170, EP-A-811336, EP-A-811337, EP-A-842620.

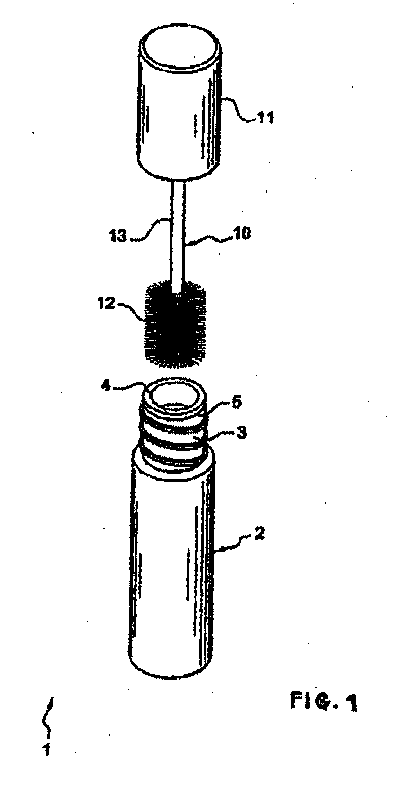

La figure 1 à laquelle il est maintenant fait référence représente un mode de réalisation préférentiel d'un ensemble de conditionnement et d'application 1 contenant une composition de revêtement des cils selon l'invention.

L'ensemble de conditionnement et d'application 1 comprend un récipient 2 sur monté d'un col fileté 3 dont un bord libre délimite une ouverture 4. Dans l'ouverture 4, est monté un organe d'essorage 5. L'ensemble 1 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient. rieur du récipient 2, le filetage du bouchon 11 vient en engagement avec le filetage du col 3 de manière à ce que le bouchon obture de manière étanche l'ouverture 4 du récipient.

Alternativement, l'applicateur peut être constitué d'un peigne comprenant généralement une pluralité de dents obtenues de moulage avec un support en matériau thermoplastique. L'applicateur peut encore être constitué d'un peigne combiné avec une brosse.

L'invention est illustrée plus en détail dans les exemples suivants.

### Exemples 1 à 6 :

On a préparé un mascara anhydre selon l'invention (exemple 1) et 5 mascaras ne faisant pas partie de l'invention (exemples 2 à 6) ayant la composition suivante, en utilisant 6 cires différentes :
- Cire 27 g
- Bentonite 5,3 g
- Carbonate de propylène 1,7 g
- Copolymère acétate de vinyle/stéarate d'allyle (65/35) (Mexomère PQ de CHIMEX) 2,2 g
- Polylaurate de vinyle (Mexomère PP de CHIMEX) 0,7 g
- Silice 0,8 g
- Pigments 3,6 g
- Conservateurs qs
- Isododécane qsp 100 g

Pour chaque composition, on a mesuré la viscosité et l'indice de consistance, et on a évalué la stabilité à 25 °C.

La mesure de la viscosité est effectuée à 25 °C avec un viscosimètre RHEOMAT RM 180 équipé d'un mobile n° 4, la mesure étant effectuée après 10 minutes de rotation du mobile (temps au bout duquel on observe une stabilisation de la viscosité et de la vitesse de rotation du mobile), à un cisaillement de 200 s⁻¹.

La mesure de l'indice de consistance est effectuée avec un texturomètre TA-TX2i par la Société RHEO, équipé d'une sonde cylindrique en inox de 12 mm de diamètre.

On remplit un récipient cylindrique (diamètre de 35 mm et profondeur de 15 mm) avec la composition de mascara à tester puis on arase la surface du produit contenu dans le récipient pour obtenir une surface bien plane du produit. La sonde cylindrique du texturomètre est déplacée à la vitesse de 10 mm.s⁻¹ puis pénètre dans le mascara contenu dans le récipient cylindrique jusqu'à une profondeur de 0,2 mm. On mesure alors la force exercée par le mascara sur la sonde, cette force correspondant à l'indice de consistance du mascara, exprimée en Pa.

La stabilité est évaluée par observation visuelle de la composition après deux semaines de stockage à 25 °C.

La mesure du collant et de la dureté de la cire est effectuée selon la méthode de mesure décrite précédemment dans la description.

On a obtenu les résultats suivants :

| **Exemple** | **Cire** | **Collant (N.s)** | **Dureté (MPa)** | **Viscosité (Pa.s)** | **Consistance (Pa)** | **Stabilité** |
|---|---|---|---|---|---|---|
| 1 | Koster K 82 P | 3,38 | 0,96 | 3,6 | 560 | oui |
| 2 | Abeille | 2,02 | 3,68 | 5,9 | 1842 | oui |
| 3 | Huile jojoba hydrogénée | 0,18 | 8,62 . | 12,8 | 1991 | oui |
| 4 | Huile ricin hydrogénée | 0,08 | 2,77 | Trop épais | 22942 | - |
| 5 | Cire d'orange⁽¹⁾ | 0,09 | 0,09 | < 1 | Trop fluide | Non 2 phases |
| 6 | Cire de lanoline oxy-propylénée (5OP)⁽²⁾ | 0,14 | 0,06 | < 1 | Trop fluide | Non 2 phases |

| | | | | | | |
|---|---|---|---|---|---|---|
| (1) vendue par la société Koster Keunen (2) EMERY 1695 de la société COGNIS | | | | | | |

On constate que la composition 1 selon l'invention est stable et présente la plus faible viscosité et la plus faible consistance. Les compositions 2 et 3 bien que stables ont une viscosité et une consistance plus élevée que clles de la composition 1. La composition 4 est trop épaisse et n'est donc pas adaptée pour être appliquée sur les cils à l'aide d'une brosse à mascara.
Les compositions 5 et 6 ne sont pas stables : elles présentent 2 phases au bout de deux semaines de stockage à 25 °C.

### Exemple 7 :

On a préparé un mascara émulsion cire-dans-eau ayant la composition suivante :
- Cire collante (Kester Wax K 82 P de la société Koster Keunen) 30 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Pigments 5,4 g
- Conservateurs qs
- Eau qsp 100 g

Ce mascara est stable après 24 heures à température ambiante. Il s'applique facilement et adhère bien sur les cils. Le mascara forme un maquillage lisse et homogène, et épaissit les cils.

### Exemple 8 :

On a préparé un mascara anhydre ayant la composition suivante :
- Cire collante (Kester Wax K 82 P de la société Koster Keunen) 30 g
- Bentonite 5,3 g
- Carbonate de propylène 1,7 g
- Copolymère acétate de vinyle/stéarate d'allyle (65/35) (Mexomère PQ de CHIMEX) 2,2 g
- Polylaurate de vinyle (Mexomère PP de CHIMEX) 0,7 g
- Stéarate de l'oligomère de l'acide 12-hydroxystéarique (SOLSPERSE 21000 de AVECIA) 0,1 g
- Silice 0,8 g
- Pigments 4,2 g
- Conservateurs qs
- Isododécane qsp 100 g

Ce mascara waterproof adhère bien sur les cils. Il confère aux cils un maquillage lisse et homogène, très séparant.

### Exemple 9 :

On a préparé un mascara anhydre ayant la composition suivante :
- Cire collante (Kester Wax K 82 P de la société Koster Keunen) 35 g
- Bentonite 5,3 g
- Carbonate de propylène 1,7 g
- Copolymère acétate de vinyle/stéarate d'allyle (65/35) (Mexomère PQ de CHIMEX) 2,2 g
- Polylaurate de vinyle (Mexomère PP de CHIMEX )0,7 g
- Stéarate de l'oligomère de l'acide 12-hydroxystéarique (SOLSPERSE 21000 de AVECIA) 0,1 g
- Silice 0,8 g
- Pigments 4,2 g
- Conservateurs qs
- Isododécane qsp 100g

Ce mascara waterproof adhère bien sur les cils. Il confère aux cils un maquillage lisse et homogène et chargeant.

### Exemple 10:

On a préparé un mascara émulsion cire-dans-eau ayant la composition suivante :
- Cire collante (Kester Wax K 82 P de la société Koster Keunen) 1.5 g
- Cire microcristalline 10 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Pigments 5,4 g
- Conservateurs qs
- Eau qsp 100 g

Ce mascara adhère bien sur les cils. Le maquillage est lisse et homogène.

### Exemple 11 :

On a préparé un mascara émulsion cire-dans-eau ayant la composition suivante :
- Cire collante (Kester Wax K 82 P de la société Koster Keunen) 25 g
- Cire de Candellila 6 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Isononanoate d'isononyle 3 g
- Pigments 5,4 g
- Conservateurs qs
- Eau qsp 100 g

Ce mascara s'applique facilement sur les cils et le maquillage des cils est lisse et homogène.

### Exemple 12 :

On a préparé un mascara émulsion cire-dans-eau ayant la composition suivante :
- Cire collante (Kester Wax K 82 P de la société Koster Keunen) 25 g
- Cire de carnauba 3 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Pigments 5,4 g
- Conservateurs qs
- Eau qsp 100 g

Ce mascara est stable après 24 heures à température ambiante. Il s'applique facilement et adhère bien sur les cils. Le mascara forme un maquillage lisse et homogène, et épaissit les cils.

### Exemple 13 :

On a préparé un mascara anhydre ayant la composition suivante :
- Cire collante (Kester Wax K 82 P de la société Koster Keunen) 17,5 g
- cire microcristalline 17,5 g
- Bentonite 5,3 g
- Carbonate de propylène 1,7 g
- Copolymère acétate de vinyle/stéarate d'allyle (65/35) (Mexomère PQ de CHIMEX) 2,2 g
- Polylaurate de vinyle (Mexomère PP de CHIMEX) 0,7 g
- Stéarate de l'oligomère de l'acide 12-hydroxystéarique (SOLSPERSE 21000 de AVECIA) 0,1 g
- Talc 0,8 g
- Pigments 4,2 g
- Conservateurs qs
- Isododécane qsp 100 g

Ce mascara waterproof adhère bien sur les cils. Il confère aux cils un maquillage lisse, homogène et chargeant.

### Exemples 14 à 16

Les mascaras anhydres suivants ont été préparés :

| | | |
|---|---|---|
| Cire collante (Kester Wax K 82 P^{®} de la société Koster Keunen) | | 27 g |
| Bentonite | | 2,66 g |
| Carbonate de propylène Copolymère acétate de vinyle/stéarate d'allyle (65/35) | | 1,7 g |
| (Mexomère PQ^{®} de CHIMEX) | | 2,2 g |
| Polylaurate de vinyle (Mexomère PP^{®} de CHIMEX) Stéarate de l'oligomère de l'acide 12-hydroxystéarique | | 0,7 g |
| (SOLSPERSE 21000^{®} de AVECIA) | | 0,1 g |
| charge à surface spécifique | | 15 g |
| Pigments | | 4,2 g |
| Conservateurs | qsp | |
| Isododécane | qsp | 100 g |

A partir de cette formulation, ont été préparés trois mascaras différents conformes à l'invention en incorporant pour chacun d'entre eux une charge spécifique.
La nature des trois charges choisies, leurs spécificités et quantités respectives sont indiquées dans le tableau 1.
La silice Sunsil 130^{®} est commercialisé par Sunjin Chemical.
Le polyméthacrylate de méthyle Jurymer MB1^{®} est commercialisé par Nihon Junyakuet la silice Sunsphère H-51^{®} est commercialisé par Asahi Glass.
Le collant est apprécié selon le protocole suivant :
On maquille une éprouvette constituée de cheveux raides (60 cheveux de 15mm de longueur) en appliquant le produit selon 30 passages successifs avec une brosse. Après un séchage d'une heure, on frotte les cheveux maquillés par un mouvement d'aller et retour avec le doigt. Le collant est apprécié qualitativement selon le dégré de cheveux collés de 1 (pas collé du tout) à 5 (très collés).
Les trois mascaras waterproof ainsi obtenus adhèrent bien sur les cils. Ils confèrent aux cils un maquillage lisse et homogène, très séparant.

### Exemple 17

On a préparé un mascara anhydre waterproof ayant la composition suivante :

| | | |
|---|---|---|
| Cire collante (Kester Wax K 82 P^{®} de la société Koster Keunen) | | 32 g |
| Palmitate de dextrine (Rheopearl KL^{®} de Chiba Flour) | | 5,3 g |
| Copolymère acétate de vinyle/stéarate d'allyle (65135) | | 2,2 g |
| (Mexomère PQ^{®} de CHIMEX) | | |
| Polylaurate de vinyle (Mexomère PP^{®} de CHIMEX) | | 0,75 g |
| Stéarate de l'oligomère de l'acide 12-hydroxystéarique | | 0,1 g |
| (SOLSPERSE 21000^{®} de AVECIA) | | |
| Silice | | 10 g |
| Talc | | 0,84 g |
| Pigments | | 4,6 g |
| Conservateurs | qs | |
| Isododécane | qsp | 100 g |

Le mascara s'applique facilement sur les cils et permet d'obtenir un maquillage épais des cils et non collant : les cils sont bien séparés.

### Exemple 18

On a préparé un mascara émulsion cire / eau ayant la composition suivante :

| | | |
|---|---|---|
| Cire collante (Kester Wax K 82 P^{®} de la société Koster Keunen) | | 25 g |
| Cire candellila | | 3 g |
| Palmitate de dextrine (Rheopearl KL^{®} de Chiba Flour) | | 6 g |
| acide stéarique | | 5,8 g |
| amino-2 methyl-2 propanediol 1,3 | | 0,5 g |
| triethanolamine | | 2,4 g |
| hydroxyethycellulose | | 0,9 g |
| Silice | | 5 g |
| Pigments | | 5,5 g |
| Conservateurs | qs | |
| Eau | qsp | 100 g |

Le mascara s'applique très facilement sur les cils et permet d'obtenir un dépôt homogène, chargeant et séparant.

**Tableau 1**

| Exemple (Formule) | Nom commercial | Nature chimique | Masse (g) | Taille de la charge µm | Surface spécifique m²/g | Collant après frottement sur éprouvettes |
|---|---|---|---|---|---|---|
| N° 1 | Sunsil 130^{®} | Silice creuse et sphérique | 15 | 6-9 | 200-260 | 1 |
| N° 2 | Jurymer MB1^{®} | polyméthacrylate de méthyle | 15 | 8-15 | 300 | 2 |
| N° 3 | Sunsphère^{®} H-51 | Microsphères de silice creuse | 15 | 5 | 770 | 1 |

## Revendications

1. Procédé cosmétique de maquillage ou de soin non thérapeutique des cils comprenant l'application sur les cils d'une composition comprenant, dans un milieu cosmétiquement acceptable, une première cire ayant un collant supérieur ou égal à 0,7 N.s et une dureté inférieure ou égale à 3,5 MPa, le collant étant mesuré à 20 °C à l'aide du texturomètre vendu sous la dénomination TA-TX2i par la société RHEO, équipé d'un mobile en polymère acrylique en forme de cône formant un angle de 45°, selon le protocole indiqué dans la description, et la dureté étant mesurée à 20 °C à l'aide du texturomètre vendu sous la dénomination TA-XT21 par la société RHEO, équipé d'un cylindre en Inox d'un diamètre de 2 mm, selon le protocole indiqué dans la description.

2. Procédé selon la revendication 1, **caractérisé par le fait que** la première cire a un collant allant de 0,7 N.s à 30 N.s.

3. Procédé selon la revendication 1 ou 2, **caractérisé par le fait que** la première cire a un collant supérieur ou égal à 1 N.s, de préférence allant de 1 N.s à 20 N.s.

4. Procédé selon l'une quelconque des revendications précédentes, **caractérisé par le fait que** la première cire a un collant supérieur ou égal à 2 N.s, de préférence allant de 2 N.s. à 10 N.s.

5. Procédé selon l'une quelconque des revendications précédentes, **caractérisé par le fait que** la première cire a une dureté allant de 0,01 à 3,5 MPa.

6. Procédé selon l'une quelconque des revendications précédentes, **caractérisé par le fait que** la première cire a une dureté allant de 0,05 MPa à 3 MPa, et de préférence allant de 0,1 MPa à 2,5 MPa.

7. Procédé selon l'une quelconque des revendications précédentes, **caractérisé par le fait que** la première cire est un (hydroxystéaroyloxy)stéarate d'alkyle en C₂₀-C₄₀.

8. Procédé selon l'une quelconque des revendications précédentes, **caractérisé par le fait que** la première cire est présente en une teneur allant de 0,5 % à 60 % en poids, par rapport au poids total de la composition, de préférence allant de 5 % à 50 % en poids, et préférentiellement allant de 10 % à 40 % en poids.

9. Procédé selon l'une quelconque des revendications précédentes, **caractérisé par le fait que** la première cire est présente en une teneur supérieure à 25 % en poids par rapport au poids total de la composition, de préférence supérieure à 27% en poids, mieux supérieure à 28% en poids, et plus préférentiellement supérieure à 30% en poids.

10. Procédé selon la revendication 7, **caractérisé par le fait que** l'(hydroxystéaroyloxy)stéarate d'alkyle en C₂₀-C₄₀ répond à la formule (I) suivante : 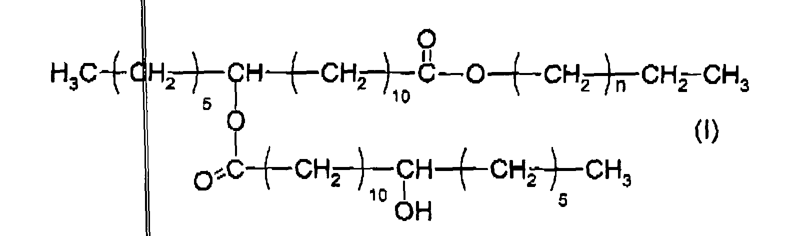 dans laquelle n est un entier allant de 18 à 38, ou un mélange de composés de formule (I).

11. Procédé selon l'une quelconque des revendications précédentes, **caractérisé par le fait que** la composition comprend une deuxième cire ayant une dureté supérieure ou égale à 6 MPa.

12. Procédé selon la revendication précédente, **caractérisé par le fait que** la deuxième cire a une dureté allant de 6 MPa à 30 MPa, de préférence allant de 7 MPa à 25 MPa, préférentiellement allant de 8 MPa à 25 MPa, mieux allant de 9 à 20 MPa et encore mieux allant de 10 MPa à 20 MPa.

13. Procédé selon la revendication 11 ou 12, **caractérisé par le fait que** la deuxième cire est choisie parmi la cire de carnauba, les cires de polyéthylène, la cire de Candelilla, l'huile de jojoba hydrogénée, le tétrastéarate de di-(triméthylol-1, 1, 1 propane), la cire obtenue par hydrogénation d'huile d'olive estérifiée avec l'alcool stéarylique.

14. Procédé selon l'une des revendications 11 à 13, **caractérisé par le fait que** la deuxième cire est présente en une teneur allant de 0,1 % à 30 % en poids, par rapport au poids total de la composition, de préférence allant de 1 % à 20 % en poids, et plus préférentiellement allant de 2 % à 10 % en poids.

15. Procédé selon l'une des revendications précédentes, **caractérisé en ce que** la composition comprend au moins un composé choisi parmi un ester de dextrine et d'acide(s) gras et/ou une charge possédant une surface spécifique BET supérieure ou égale à 100 m²/g.

16. Procédé selon la revendication 15, **caractérisé en ce que** l'ester de dextrine et d'acide(s) gras répond à la formule (II) : 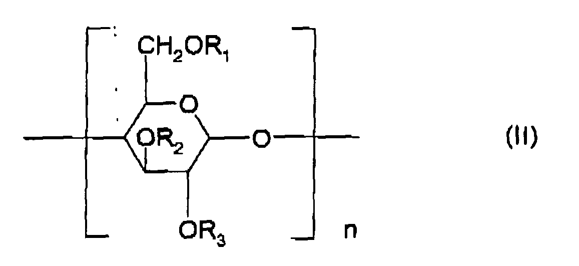 dans laquelle :
n est un entier allant de 3 à 200, notamment allant de 20 à 150, et en particulier allant de 25 à 50,
les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant de 5 à 29 atomes de carbone sous réserve qu'au moins un desdits radicaux R_{1,}
R₂ ou R₃ est différent de l'hydrogène.

17. Procédé selon la revendication 16, **caractérisé en ce qu'**au moins deux desdits radicaux R₁, R₂ ou R₃ sont identiques et différents de l'hydrogène.

18. Procédé selon la revendication 16 ou 17, **caractérisé en ce que** l'ester de dextrine et d'acide gras a un degré de substitution inférieur ou égal à 2,5 sur la base d'une unité glucose, notamment variant de 1,5 à 2,5, et en particulier de 2 à 2,5.

19. Procédé selon la revendication 16 ou 17, **caractérisé en ce que** n varie de 25 à 50 et notamment est égal à 38 dans la formule (II).

20. Procédé selon l'une quelconque des revendications 16 à 19, **caractérisé en ce que** le groupement acyle est choisi parmi les radicaux caprylyle, caproyle, lauroyle, myristyle, palmityle, stéaryle, elcosanyle, docosanoyle, isovaléryle, éthyl-2 butyryle,éthyl méthylacétyle, isoheptanyle, éthyl-2 hexanyle, isononanyle, isodécanyle, isotridécanyle, isomyristyle, isopalmityle, isostéaryle, Ioohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

21. Procédé selon l'une quelconque des revendications 16 à 20, **caractérisé en ce que** l'ester de dextrine et d'acide(s) gras comprend au moins le palmitate de dextrine.

22. Procédé selon l'une quelconque des revendications 16 à 21, **caractérisé en ce que** le poids moléculaire moyen en poids de l'ester de dextrine et d'acide(s) gras varie de 10 000 à 150 000, notamment de 12 000 à 100 000, voire de 15 000 à 80 000.

23. Procédé selon l'une quelconque des 16 à 22, **caractérisé en ce que** ledit ester de dextrine et d'acide(s) gras est présent à une quantité allant de 0,1 à 20 %, en particulier de 0,5 à 15 % en poids et notamment de 1 à 10 % en poids par rapport au poids total de la composition.

24. Procédé selon l'une quelconque des revendications 16 à 23, **caractérisé en ce que** l'ester de dextrine et d'acide(s) gras et la cire collante sont présents à une teneur telle que le rapport pondéral de la première cire par rapport audit ester varie de 350 à 0,1, en particulier de 100 à 0,5, notamment de 50 à 1, voire de 15 à 2.

25. Procédé selon l'une quelconque des revendications 16 à 24, **caractérisé en ce que** l'ester de dextrine et d'acide(s) gras et l'(hydroxystearoyloxy)stéarate d'alkyle sont présents en une teneur telle que le rapport pondéral de la première cire par rapport audit ester varie de 350 à 0,1, en particulier de 100 à 0,5, notamment de 50 à 1, voire de 15 à 2.

26. Procédé selon l'une des revendications 1 à 15, **caractérisé en ce que** la composition comprend au moins une charge possédant une surface spécifique variant de 100 à 5000 m²/g notamment de 150 à 1000 m²/g, et en particulier de 200 à 800 m²/g.

27. Procédé selon la revendication 15 ou 26, **caractérisé en ce que** la charge à surface spécifique est choisie parmi les charges organiques, minérales, et leurs mélanges.

28. Procédé selon la revendication 27, **caractérisé en ce que** la charge à surface spécifique organique est choisie parmi les cires de polyoléfines et notamment les cires de polyéthylène et les charges polymériques du type polyméthylméthacrylate ou poytétrafluoroéthylène.

29. Procédé selon la revendication 27, **caractérisé en ce que** la charge à surface spécifique minérale est choisie parmi les silices, les alumines, les silicates, et les aluminosilicates.

30. Procédé selon la revendication 15 ou l'une des revendications 26 à 29, **caractérisé en ce que** les particules composant la charge ont une taille moyenne variant de 0,01 à 100 µm, notamment de 0,1 à 50 µm et en particulier de 1 à 20 µm.

31. Procédé selon la revendication 15 ou l'une des revendications 26 à 30, **caractérisé en ce que** les particules composant la charge à surface spécifique sont creuses.

32. Procédé selon la revendication 15 ou l'une des revendications 26 à 31, **caractérisé en ce que** les particules composant la charge à surface spécifique sont des microsphères de silice creuse.

33. Procédé selon la revendication 15 ou l'une des revendications 28 à 34, **caractérisé en ce que** le rapport pondéral de la première cire par rapport à la charge à surface spécifique varie de 350 à 0,1, notamment de 100 à 0,5, et en particulier de 50 à 0,8 et mieux de 30 à 1.

34. Procédé selon la revendication 15 ou l'une des revendications 26 à 33, **caractérisé en ce que** la composition contient de 0,1 à 25 % de charge à surface spécifique, notamment de 0,5 à 20 %, et en particulier de 1 à 15 % en poids par rapport au poids total de la composition,

35. Procédé selon l'une quelconque des revendications précédentes, **caractérisé par le fait que** la composition comprend une phase aqueuse.

36. Procédé selon l'une quelconque des revendications précédentes, **caractérisé par le fait que** la composition comprend une phase aqueuse formée d'eau ou d'un mélange d'eau et de solvant organique miscible à l'eau.

37. Procédé selon la revendication précédente, **caractérisé par le fait que** le solvant organique miscible à l'eau est choisi parmi les monoalcools inférieurs ayant de 1 à 5 atomes de carbone, les glycols ayant de 2 à 8 atomes de carbone, les cétones en C₃-C₄, les aldéhydes en C₂-C₄.

38. Procédé selon l'une des revendications 36 ou 37, **caractérisé par le fait que** la phase aqueuse a est présente en une teneur allant de 1 % à 95 % en poids, par rapport au poids total de la composition, de préférence allant de 3 % à 80 % en poids, et préférentiellement allant de 5 % à 60 % en poids.

39. Procédé selon l'une quelconque des revendications précédentes, **caractérisé par le fait que** la composition comprend une huile volatile.

40. Procédé selon la revendication précédente, **caractérisé par le fait que** l'huile volatile est choisies parmi les huiles hydrocarbonées, les huiles siliconées, ou leurs mélanges.

41. Procédé selon l'une des revendications 39 ou 40, **caractérisé par le fait que** l'huile volatile est présente en une teneur allant de 0,1 % à 98 % en poids, par rapport au poids total de la composition, de préférence allant de 1 % à 65 % en poids.

42. Procédé selon l'une quelconque des revendications précédentes, **caractérisé par le fait que** la composition comprend une huile non volatile.

43. Procédé selon la revendication précédente, **caractérisé par le fait que** l'huile non volatile est présente en une teneur allant de 0,1 % à 30 % en poids, de préférence de 0,1 % à 20 % en poids, par rapport au poids total de la composition, et mieux de 0.1 % à 10 % en poids.

44. Procédé selon l'une quelconque des revendications précédentes, **caractérisé par le fait que** la composition comprend un polymère filmogène.

45. Procédé selon la revendication précédente, **caractérisé par le fait que** le polymère filmogène est présent en une teneur en matières sèches allant de 0,1 % à 60 % en poids par rapport au poids total de la composition, de préférence allant de 0,5 % à 40% en poids, et préférentiellement allant de 1 % à 30 % en poids.

46. Procédé selon l'une quelconque des revendications précédentes, **caractérisé par le fait que** la composition comprend une cire additionnelle.

47. Procédé selon la revendication précédente, **caractérisé par le fait que** la cire additionnelle est présente dans la composition en une teneur allant de 0,1 % à 50 % en poids, par rapport au poids total de la composition, de préférence de 0,5 % à 30 % en poids, et mieux de 1 % à 20 % en poids.

48. Procédé selon l'une quelconque des revendications précédentes, **caractérisé par le fait que** la composition comprend un tensioactif.

49. Procédé selon l'une quelconque des revendications précédentes, **caractérisé par le fait que** la composition comprend un additif choisi parmi les matières colorantes, les antioxydants, les charges, les corps gras pâteux, les conservateurs, les parfums, les neutralisants, les épaississants, les vitamines, les agents de coalescence, les plastifiants, et leurs mélanges.

50. Procédé selon l'une quelconque des revendications précédentes, **caractérisé par le fait que** la composition ne contient pas de filtre UV.

## Claims

1. Cosmetic process for making up or for the non-therapeutic care of the eyelashes, comprising the application to the eyelashes of a composition comprising, in a cosmetically acceptable medium, a first wax with a tack of greater than or equal to 0.7 N.s and a hardness of less than or equal to 3.5 MPa, the tack being measured at 20°C using a texturometer sold under the name TA-TX2i by the company Rheo, equipped with a cone-shaped acrylic polymer spindle forming an angle of 45°, according to the protocol indicated in the description, and the hardness being measured at 20°C using a texturometer sold under the name TA-XT2i by the company Rheo, equipped with a stainless-steel cylinder 2 mm in diameter, according to the protocol indicated in the description.

2. Process according to Claim 1, **characterized in that** the first wax has a tack ranging from 0.7 N.s to 30 N.s.

3. Process according to Claim 1 or 2, **characterized in that** the first wax has a tack of greater than or equal to 1 N.s, preferably ranging from 1 N.s to 20 N.s.

4. Process according to any one of the preceding claims, **characterized in that** the first wax has a tack of greater than or equal to 2 N.s, preferably ranging from 2 N.s to 10 N.s.

5. Process according to any one of the preceding claims, **characterized in that** the first wax has a hardness ranging from 0.01 to 3.5 MPa.

6. Process according to any one of the preceding claims, **characterized in that** the first wax has a hardness ranging from 0.05 MPa to 3 MPa and preferably ranging from 0.1 MPa to 2.5 MPa.

7. Process according to any one of the preceding claims, **characterized in that** the first wax is a C₂₀-C₄₀ alkyl (hydroxystearoyloxy)stearate.

8. Process according to any one of the preceding claims, **characterized in that** the first wax is present in a content ranging from 0.5% to 60% by weight, preferably ranging from 5% to 50% by weight and preferentially ranging from 10% to 40% by weight relative to the total weight of the composition.

9. Process according to any one of the preceding claims, **characterized in that** the first wax is present in a content of greater than 25% by weight, preferably greater than 27% by weight, better still greater than 28% by weight and more preferentially greater than 30% by weight relative to the total weight of the composition.

10. Process according to Claim 7, **characterized in that** the C₂₀-C₄₀ alkyl (hydroxystearoyloxy)stearate corresponds to formula (I) below: 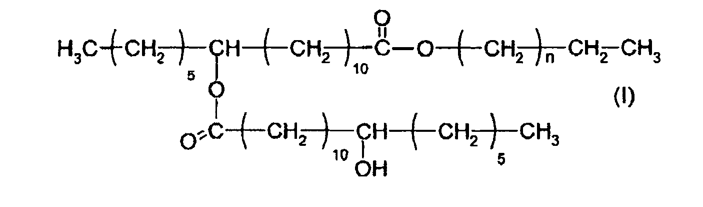 in which n is an integer ranging from 18 to 38, or a mixture of compounds of formula (I).

11. Process according to any one of the preceding claims, **characterized in that** the composition comprises a second wax with a hardness of greater than or equal to 6 MPa.

12. Process according to the preceding claim, **characterized in that** the second wax has a hardness ranging from 6 MPa to 30 MPa, preferably ranging from 7 MPa to 25 MPa, preferentially ranging from 8 MPa to 25 MPa, better still ranging from 9 MPa to 20 MPa and even better still ranging from 10 MPa to 20 MPa.

13. Process according to Claim 11 or 12, **characterized in that** the second wax is chosen from carnauba wax, polyethylene waxes, candelilla wax, hydrogenated jojoba oil, bis(1,1,1-trimethylolpropane) tetrastearate, and the wax obtained by hydrogenation of olive oil esterified with stearyl alcohol.

14. Process according to one of Claims 11 to 13, **characterized in that** the second wax is present in a content ranging from 0.1% to 30% by weight, preferably ranging from 1% to 20% by weight and more preferentially ranging from 2% to 10% by weight relative to the total weight of the composition.

15. Process according to one of the preceding claims, **characterized in that** the composition comprises at least one compound chosen from a fatty acid ester of dextrin and/or a filler with a BET specific surface area of greater than or equal to 100 m²/g.

16. Process according to Claim 15, **characterized in that** the fatty acid ester of dextrin corresponds to formula (II): 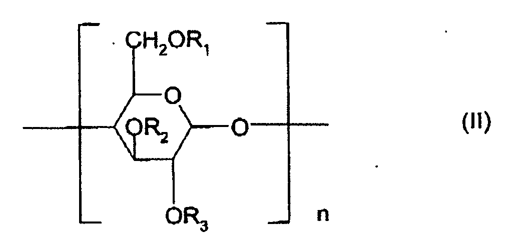 in which:
n is an integer ranging from 3 to 200, especially ranging from 20 to 150 and in particular ranging from 25 to 50,
the radicals R₁, R₂ and R₃, which may be identical or different, are chosen from hydrogen and an acyl group (R-CO-) in which the radical R is a linear or branched, saturated or unsaturated hydrocarbon-based group containing from 5 to 29 carbon atoms, with the proviso that at least one of the said radicals R₁, R₂ or R₃ is other than hydrogen.

17. Process according to Claim 16, **characterized in that** at least two of the said radicals R₁, R₂ or R₃ are identical and other than hydrogen.

18. Process according to Claim 16 or 17, **characterized in that** the fatty acid ester of dextrin has a degree of substitution of less than or equal to 2.5, especially ranging from 1.5 to 2.5 and in particular from 2 to 2.5, on the basis of one glucose unit.

19. Process according to Claim 16 or 17, **characterized in that** n ranges from 25 to 50 and is especially equal to 38 in formula (II).

20. Process according to any one of Claims 16 to 19, **characterized in that** the acyl group is chosen from the radicals caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitoleyl, oleyl, elaidyl, eicosenyl, sorbyl, linoleyl, linolenyl, punicyl, arachidonyl and stearolyl, and mixtures thereof.

21. Process according to any one of Claims 16 to 20, **characterized in that** the fatty acid ester of dextrin comprises at least dextrin palmitate.

22. Process according to any one of Claims 16 to 21, **characterized in that** the weight-average molecular weight of the fatty acid ester of dextrin ranges from 10 000 to 150 000, especially from 12 000 to 100 000 or even from 15 000 to 80 000.

23. Process according to any one of Claims 16 to 22, **characterized in that** the said fatty acid ester of dextrin is present in an amount ranging from 0.1% to 20% by weight, in particular from 0.5% to 15% by weight and especially from 1% to 10% by weight relative to the total weight of the composition.

24. Process according to any one of Claims 16 to 23, **characterized in that** the fatty acid ester of dextrin and the tacky wax are present in a content such that the weight ratio of the first wax relative to the said ester ranges from 350 to 0.1, in particular from 100 to 0.5 and especially from 50 to 1, or even from 15 to 2.

25. Process according to any one of Claims 16 to 24, **characterized in that** the fatty acid ester of dextrin and the alkyl (hydroxystearoyloxy) stearate are present in a content such that the weight ratio of the first wax relative to the said ester ranges from 350 to 0.1, in particular from 100 to 0.5 and especially from 50 to 1, or even from 15 to 2.

26. Process according to one of Claims 1 to 15, **characterized in that** the composition comprises at least one filler with a specific surface area ranging from 100 to 5000 m²/g, especially from 150 to 1000 m²/g and in particular from 200 to 800 m²/g.

27. Process according to Claim 15 or 26, **characterized in that** the filler with a specific surface area is chosen from organic and mineral fillers, and mixtures thereof.

28. Process according to Claim 27, **characterized in that** the organic filler with a specific surface area is chosen from polyolefin waxes and especially polyethylene waxes and polymeric fillers of the polymethyl methacrylate or polytetrafluoroethylene type.

29. Process according to Claim 27, **characterized in that** the mineral filler with a specific surface area is chosen from silicas, aluminas, silicates and aluminosilicates.

30. Process according to Claim 15 or one of Claims 26 to 29, **characterized in that** the particles of which the filler is composed have a mean size ranging from 0.01 to 100 µm, especially from 0.1 to 50 µm and in particular from 1 to 20 µm.

31. Process according to Claim 15 or one of Claims 26 to 30, **characterized in that** the particles of which the filler with a specific surface area is composed are hollow.

32. Process according to Claim 15 or one of Claims 26 to 31, **characterized in that** the particles of which the filler with a specific surface area is composed are hollow silica microspheres.

33. Process according to Claim 15 or one of Claims 28 to 32, **characterized in that** the weight ratio of the first wax relative to the filler with a specific surface area ranges from 350 to 0.1, especially from 100 to 0.5, in particular from 50 to 0.8 and better still from 30 to 1.

34. Process according to Claim 15 or one of Claims 26 to 33, **characterized in that** the composition contains from 0.1% to 25%, especially from 0.5% to 20% and in particular from 1% to 15% by weight of filler with a specific surface area relative to the total weight of the composition.

35. Process according to any one of the preceding claims, **characterized in that** the composition comprises an aqueous phase.

36. Process according to any one of the preceding claims, **characterized in that** the composition comprises an aqueous phase formed from water or from a mixture of water and of water-miscible organic solvent.

37. Process according to the preceding claim, **characterized in that** the water-miscible organic solvent is chosen from lower monoalcohols containing from 1 to 5 carbon atoms, glycols containing from 2 to 8 carbon atoms, C₃-C₄ ketones and C₂-C₄ aldehydes.

38. Process according to either of Claims 36 and 37, **characterized in that** the aqueous phase is present in a content ranging from 1% to 95% by weight, preferably ranging from 3% to 80% by weight and preferentially ranging from 5% to 60% by weight relative to the total weight of the composition.

39. Process according to any one of the preceding claims, **characterized in that** the composition comprises a volatile oil.

40. Process according to the preceding claim, **characterized in that** the volatile oil is chosen from hydrocarbon-based oils and silicone oils, or mixtures thereof.

41. Process according to either of Claims 39 and 40, **characterized in that** the volatile oil is present in a content ranging from 0.1% to 98% by weight and preferably ranging from 1% to 65% by weight relative to the total weight of the composition.

42. Process according to any one of the preceding claims, **characterized in that** the composition comprises a non-volatile oil.

43. Process according to the preceding claim, **characterized in that** the non-volatile oil is present in a content ranging from 0.1% to 30% by weight, preferably from 0.1% to 20% by weight and better still from 0.1% to 10% by weight relative to the total weight of the composition.

44. Process according to any one of the preceding claims, **characterized in that** the composition comprises a film-forming polymer.

45. Process according to the preceding claim, **characterized in that** the film-forming polymer is present in a solids content ranging from 0.1% to 60% by weight, preferably ranging from 0.5% to 40% by weight and preferentially ranging from 1% to 30% by weight relative to the total weight of the composition.

46. Process according to any one of the preceding claims, **characterized in that** the composition comprises an additional wax.

47. Process according to the preceding claim, **characterized in that** the additional wax is present in the composition in a content ranging from 0.1% to 50% by weight, preferably from 0.5% to 30% by weight and better still from 1% to 20% by weight relative to the total weight of the composition.

48. Process according to any one of the preceding claims, **characterized in that** the composition comprises a surfactant.

49. Process according to any one of the preceding claims, **characterized in that** the composition comprises an additive chosen from dyestuffs, antioxidants, fillers, pasty fatty substances, preserving agents, fragrances, neutralizers, thickeners, vitamins, coalescers and plasticizers, and mixtures thereof.

50. Process according to any one of the preceding claims, **characterized in that** the composition does not contain any UV-screening agent.

## Patentansprüche

1. Kosmetisches Verfahren zum Schminken und zur nichttherapeutischen Pflege der Wimpern, das das Auftragen einer Zusammensetzung auf die Wimpern umfasst, die in einem kosmetisch akzeptablen Medium ein erstes Wachs mit einer Klebrigkeit von 0,7 N.s oder darüber und einer Härte von 3,5 MPa oder darunter enthält, wobei die Klebrigkeit bei 20 °C nach der in der Beschreibung angegebenen Vorgehensweise mit einem Texturmessgerät gemessen wird, das unter der Bezeichnung TA-TX2i von der Firma RHEO erhältlich ist und das mit einem beweglichen Teil aus einem Acrylpolymer in Form eines Kegels, der einen Winkel von 45° bildet, ausgestattet ist, und die Härte bei 20 °C nach der in der Beschreibung angegebenen Vorgehensweise mit einem Texturmessgerät gemessen wird, das unter der Bezeichnung TA-XT2i von der Firma RHEO verkauft wird und mit einem Edelstahlzylinder mit einem Durchmesser von 2 mm ausgestattet ist.

2. Verfahren nach Anspruch 1, **dadurch gekennzeichnet, dass** das erste Wachs eine Klebrigkeit von 0,7 bis 30 N.s aufweist.

3. Verfahren nach Anspruch 1 oder 2, **dadurch gekennzeichnet, dass** das erste Wachs eine Klebrigkeit von 1 N·s oder darüber und vorzugsweise 1 bis 20 N·s aufweist.

4. Verfahren nach einem der vorhergehenden Ansprüche, **dadurch gekennzeichnet, dass** das erste Wachs eine Klebrigkeit von 2 N·s oder darüber und vorzugsweise 2 bis 10 N·s aufweist.

5. Verfahren nach einem der vorhergehenden Ansprüche, **dadurch gekennzeichnet, dass** das erste Wachs eine Härte von 0,01 bis 3,5 MPa besitzt.

6. Verfahren nach einem der vorhergehenden Ansprüche, **dadurch gekennzeichnet, dass** das erste Wachs eine Härte von 0,05 bis 3 MPa und vorzugsweise 0,1 bis 2,5 MPa aufweist.

7. Verfahren nach einem der vorhergehenden Ansprüche, **dadurch gekennzeichnet, dass** das erste Wachs ein Alkyl(C₂₀₋₄₀)-(hydroxystearoyloxy)stearat ist.

8. Verfahren nach einem der vorhergehenden Ansprüche, **dadurch gekennzeichnet, dass** das erste Wachs in einer Menge von 0,5 bis 60 Gew.-%, bezogen auf das Gesamtgewicht der Zusammensetzung, vorzugsweise 5 bis 50 Gew.-% und bevorzugt 10 bis 40 Gew.-% enthalten ist.

9. Verfahren nach einem der vorhergehenden Ansprüche, **dadurch gekennzeichnet, dass** das erste Wachs in einer Menge über 25 Gew.-%, bezogen auf das Gesamtgewicht der Zusammensetzung, vorzugsweise über 27 Gew.-%, besser über 28 Gew.-% und besonders bevorzugt über 30 Gew.-% enthalten ist.

10. Verfahren nach Anspruch 7, **dadurch gekennzeichnet, dass** das Alkyl(C₂₀₋₄₀)-(hydroxystearoyloxy)stearat der folgenden Formel (I) entspricht: 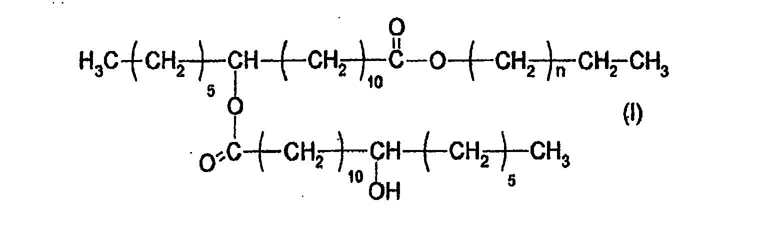 worin n eine ganze Zahl von 18 bis 38 bedeutet, oder einem Gemisch von Verbindungen der Formel (I).

11. Verfahren nach einem der vorhergehenden Ansprüche, **dadurch gekennzeichnet, dass** sie ein zweites Wachs mit einer Härte von 6 MPa oder darüber enthält.

12. Verfahren nach dem vorhergehenden Anspruch, **dadurch gekennzeichnet, dass** das zweite Wachs eine Härte von 6 bis 30 MPa, vorzugsweise 7 bis 25 MPa, bevorzugt 8 bis 25 MPa, besser 9 bis 20 MPa und noch besser 10 bis 20 MPa aufweist.

13. Verfahren nach Anspruch 11 oder 12, **dadurch gekennzeichnet, dass** das zweite Wachs unter Carnaubawachs, Polyethylenwachsen, Candilillawachs, hydriertem Jojobaöl, Di(1,1,1-trimethylolpropan)tetrastearat, einem durch Hydrierung von Olivenöl erhaltenen, mit Stearylalkohol veresterten Wachs ausgewählt ist.

14. Zusammensetzung nach einem der Ansprüche 11 bis 13, **dadurch gekennzeichnet, dass** das zweite Wachs in einer Menge von 0,1 bis 30 Gew.-%, bezogen auf das Gesamtgewicht der Zusammensetzung, vorzugsweise 1 bis 20 Gew.-% und noch bevorzugter 2 bis 10 Gew.-% enthalten ist.

15. Verfahren nach einem der vorhergehenden Ansprüche, **dadurch gekennzeichnet, dass** sie mindestens eine Verbindung enthält, die unter einem Ester aus Dextrin und einer oder mehreren Fettsäuren und/oder einem Füllstoff mit einer spezifischen BET-Oberfläche von 100 m²/g oder darüber ausgewählt ist.

16. Verfahren nach Anspruch 15, **dadurch gekennzeichnet, dass** der Ester aus Dextrin und Fettsäure(n) der Formel (II) entspricht: 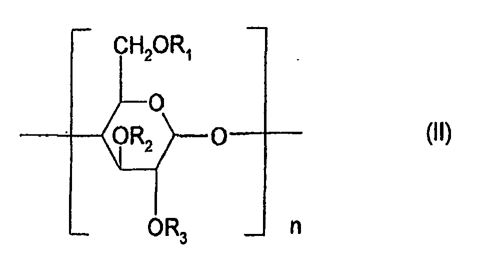 worin bedeuten:
n ist eine ganze Zahl von 3 bis 200, insbesondere 20 bis 150 und besonders 25 bis 50,
die Gruppen R₁, R₂ und R₃, die gleich oder verschieden sind, sind unter Wasserstoff oder einer Acylgruppe (R-CO-) ausgewählt, worin die Gruppe R eine geradkettige oder verzweigte, gesättigte oder ungesättigte Kohlenwasserstoffgruppe mit 5 bis 29 Kohlenstoffatomen ist, mit der Maßgabe, dass mindestens eine der Gruppen R₁, R₂ oder R₃ von Wasserstoff verschieden ist.

17. Verfahren nach Anspruch 16, **dadurch gekennzeichnet, dass** mindestens zwei Gruppen R₁, R₂ oder R₃ identisch und von Wasserstoff verschieden sind.

18. Verfahren nach Anspruch 16 oder 17, **dadurch gekennzeichnet, dass** der Dextrinfettsäureester einen Substitutionsgrad von höchstens 2,5 auf der Basis einer Glucoseeinheit und insbesondere im Bereich von 1,5 bis 2,5 und besonders 2 bis 2,5 auf weist.

19. Verfahren nach Anspruch 16 oder 17, **dadurch gekennzeichnet, dass** in der Formel (II) n im Bereich von 25 bis 50 liegt und insbesondere 38 beträgt.

20. Verfahren nach einem der Ansprüche 16 bis 19, **dadurch gekennzeichnet, dass** die Acylgruppe unter den Gruppen Caprylyl, Caproyl, Lauroyl, Myristyl, Palmityl, Stearyl, Eicosanyl, Docosanoyl, Isovaleryl, 2-Ethylbutyryl, Ethylmethylacetyl, Isoheptanyl, 2-Ethylhexanyl, Isononanyl, Isodecanyl, Isotridecanyl, Isomyristyl, Isopalmityl, Isostearyl, Isohexanyl, Decenyl, Dodecenyl, Tetradecenyl, Myristyl, Hexadecenoyl, Palmitoleyl, Oleyl, Elaidyl, Eicosenyl, Sorbyl, Linoleyl, Linolenyl, Punicyl, Arachidonyl, Stearoyl und deren Gemischen ausgewählt ist.

21. Verfahren nach einem der Ansprüche 16 bis 20, **dadurch gekennzeichnet, dass** der Ester aus Dextrin und einer oder mehreren Fettsäuren zumindest das Dextrinpalmitat umfasst.

22. Verfahren nach einem der Ansprüche 16 bis 21, **dadurch gekennzeichnet, dass** die gewichtsmittlere Molmasse des Esters aus Dextrin und Fettsäure(n) im Bereich von 10.000 bis 150.000, insbesondere 12.000 bis 100.000 und besonders 15.000 bis 80.000 liegt.

23. Verfahren nach einem der Ansprüche 16 bis 22, **dadurch gekennzeichnet, dass** der Ester aus Dextrin und Fettsäure(n) in einer Menge von 0,1 bis 20 Gew.-%, besonders 0,5 bis 15 Gew.-% und insbesondere 1 bis 10 Gew.-%, bezogen auf das Gesamtgewicht der Zusammensetzung, enthalten ist.

24. Verfahren nach einem der Ansprüche 16 bis 23, **dadurch gekennzeichnet, dass** der Ester aus Dextrin und Fettsäure(n) und das klebrige Wachs in einer solchen Menge enthalten sind, dass das Gewichtsverhältnis des ersten Wachses und des Esters im Bereich von 350 bis 0,1, insbesondere 100 bis 0,5, besonders 50 bis 1 und speziell 15 bis 2 liegt.

25. Verfahren nach einem der Ansprüche 16 bis 24, **dadurch gekennzeichnet, dass** der Ester aus Dextrin und Fettsäure(n) und das Alkyl(hydroxystearyloxy)stearat in einer solchen Menge enthalten sind, dass das Gewichtsverhältnis des ersten Wachses und des Esters im Bereich von 350 bis 0,1, insbesondere 100 bis 0,5, besonders 50 bis 1 und speziell 15 bis 2 liegt.

26. Verfahren nach einem der Ansprüche 1 bis 15, **dadurch gekennzeichnet, dass** die Zusammensetzung mindestens einen Füllstoff enthält, der eine spezifische Oberfläche besitzt, die im Bereich von 100 bis 5.000 m²/g, insbesondere 150 bis 1.000 m²/g und besonders 200 bis 800 m²/g liegt.

27. Verfahren nach Anspruch 15 oder 26, **dadurch gekennzeichnet, dass** der Füllstoff mit spezieller Oberfläche unter den organischen Füllstoffen, anorganischen Füllstoffen und deren Gemischen ausgewählt ist.

28. Verfahren nach Anspruch 27, **dadurch gekennzeichnet, dass** der organische Füllstoff mit spezieller Oberfläche unter den Polyolefinwachsen und besonders den Polyethylenwachsen und den polymeren Füllstoffen vom Typ Polymethylmethacrylat oder Polytetrafluorethylen ausgewählt ist.

29. Verfahren nach Anspruch 27, **dadurch gekennzeichnet, dass** der anorganische Füllstoff mit spezieller Oberfläche unter den Kieselsäuren, Aluminiumoxiden, Silicaten und Aluminosilicaten ausgewählt ist.

30. Verfahren nach Anspruch 15 oder nach einem der Ansprüche 26 bis 29, **dadurch gekennzeichnet, dass** die Partikel, die den Füllstoff bilden, eine mittlere Größe von 0,01 bis 100 µm, insbesondere 0,1 bis 50 µm und besonders 1 bis 20 µm besitzen.

31. Verfahren nach Anspruch 15 oder nach einem der Ansprüche 26 bis 30, **dadurch gekennzeichnet, dass** die Partikel, die den Füllstoff mit spezieller Oberfläche bilden, hohl sind.

32. Verfahren nach Anspruch 15 oder nach einem der Ansprüche 26 bis 31, **dadurch gekennzeichnet, dass** die Partikel, die den Füllstoff mit spezieller Oberfläche bilden, Siliciumoxid-Mikrohohlkugeln sind.

33. Verfahren nach Anspruch 15 oder einem der Ansprüche 28 bis 34, **dadurch gekennzeichnet, dass** das Gewichtsverhältnis des ersten Wachses und des Füllstoffs mit spezieller Oberfläche im Bereich von 350 bis 0,1, insbesondere 100 bis 0,5 und besonders 50 bis 0,8 und besser 30 bis 1 liegt.

34. Verfahren nach Anspruch 15 oder einem der Ansprüche 26 bis 33, **dadurch gekennzeichnet, dass** sie 0,1 bis 25 % Füllstoff mit spezieller Oberfläche, insbesondere 0,5 bis 20 % und besonders 1 bis 15 Gew.-%, bezogen auf das Gesamtgewicht der Zusammensetzung, enthält.

35. Verfahren nach einem der vorhergehenden Ansprüche, **dadurch gekennzeichnet, dass** die Zusammensetzung eine wässerige Phase umfasst.

36. Verfahren nach einem der vorhergehenden Ansprüche, **dadurch gekennzeichnet, dass** die Zusammensetzung eine wässerige Phase enthält, die aus Wasser oder einem Gemisch von Wasser und einem mit Wasser mischbaren organischen Lösungsmittel gebildet ist.

37. Verfahren nach dem vorhergehenden Anspruch, **dadurch gekennzeichnet, dass** das mit Wasser mischbare organische Lösungsmittel unter den niederen Monoalkoholen mit 1 bis 5 Kohlenstoffatomen, Glycolen mit 2 bis 8 Kohlenstoffatomen, Ketonen mit 3 bis 4 Kohlenstoffatomen und Aldehyden mit 2 bis 4 Kohlenstoffatomen ausgewählt ist.

38. Verfahren nach Anspruch 36 oder 37, **dadurch gekennzeichnet, dass** die wässerige Phase in einer Menge von 1 bis 95 Gew.-%, bezogen auf das Gesamtgewicht der Zusammensetzung, vorzugsweise 3 bis 80 Gew.-% und noch bevorzugter 5 bis 60 Gew.-% enthalten ist.

39. Verfahren nach einem der vorhergehenden Ansprüche, **dadurch gekennzeichnet, dass** sie ein flüchtiges Öl enthält.

40. Verfahren nach dem vorhergehenden Anspruch, **dadurch gekennzeichnet, dass** das flüchtige Öl unter den Kohlenwasserstoffölen, Siliconölen oder deren Gemischen ausgewählt ist.

41. Verfahren nach einem der Ansprüche 39 oder 40, **dadurch gekennzeichnet, dass** das flüchtige Öl in einer Menge von 0,1 bis 98 Gew.-%, bezogen auf das Gesamtgewicht der Zusammensetzung, und vorzugsweise 1 bis 65 Gew.-% enthalten ist.

42. Verfahren nach einem der vorhergehenden Ansprüche, **dadurch gekennzeichnet, dass** sie ein nichtflüchtiges Öl enthält.

43. Verfahren nach dem vorhergehenden Anspruch, **dadurch gekennzeichnet, dass** das nichtflüchtige Öl in einer Menge von 0,1 bis 30 Gew.-%, vorzugsweise 0,1 bis 20 Gew.-%, bezogen auf das Gesamtgewicht der Zusammensetzung, und noch besser 0,1 bis 10 Gew.-% enthalten ist.

44. Verfahren nach einem der vorhergehenden Ansprüche, **dadurch gekennzeichnet, dass** sie ein filmbildendes Polymer enthält.

45. Verfahren nach dem vorhergehenden Anspruch, **dadurch gekennzeichnet, dass** das filmbildende Polymer in einem Trockensubstanzgehalt von 0,1 bis 60 Gew.-%, bezogen auf das Gesamtgewicht der Zusammensetzung, vorzugsweise 0,5 bis 40 Gew.-% und noch bevorzugter 1 bis 30 Gew.-% enthalten ist.

46. Verfahren nach einem der vorhergehenden Ansprüche, **dadurch gekennzeichnet, dass** die Zusammensetzung ein zusätzliches Wachs enthält.

47. Verfahren nach dem vorhergehenden Anspruch, **dadurch gekennzeichnet, dass** das zusätzliche Wachs in der Zusammensetzung in einer Menge von 0,1 bis 50 Gew.-%, bezogen auf das Gesamtgewicht der Zusammensetzung, vorzugsweise 0,5 bis 30 Gew.-% und noch besser 1 bis 20 Gew.-% enthalten ist.

48. Verfahren nach einem der vorhergehenden Ansprüche, **dadurch gekennzeichnet, dass** die Zusammensetzung einen grenzflächenaktiven Stoff enthält.

49. Verfahren nach einem der vorhergehenden Ansprüche, **dadurch gekennzeichnet, dass** die Zusammensetzung einen Zusatzstoff enthält, der unter den Farbmitteln, Antioxidantien, Füllstoffen, pastösen Fettsubstanzen, Konservierungsmitteln, Parfums, Neutralisationsmitteln, Verdickungsmitteln, Vitaminen, Koaleszenzmitteln, Weichmachern und deren Gemischen ausgewählt ist.

50. Verfahren nach einem der vorhergehenden Ansprüche, **dadurch gekennzeichnet, dass** die Zusammensetzung kein UV-Filter enthält.
